(19) Europäisches Patentamt
European Patent Office
Office européen des brevets

(11) Numéro de publication : **0 022 022**
**B1**

(12) **FASCICULE DE BREVET EUROPÉEN**

(45) Date de publication du fascicule du brevet : **11.05.83**

(51) Int. Cl.³ : **C 07 C 143/155**, C 07 C 101/24, C 07 C 129/08, A 61 K 31/185

(21) Numéro de dépôt : **80400950.4**

(22) Date de dépôt : **25.06.80**

(54) **Nouveaux dérivés acylés de la taurine et leurs sels et procédé pour leur préparation.**

(30) Priorité : **29.06.79 FR 7917021**

(43) Date de publication de la demande : **07.01.81 Bulletin 81/01**

(45) Mention de la délivrance du brevet : **11.05.83 Bulletin 83/19**

(84) Etats contractants désignés : **BE DE GB IT NL**

(56) Documents cités :
**FR A 2 412 523**

**CHEMICAL ABSTRACTS, vol. 75, nr. 19, 8th november 1971, page 71, ref. 116449f Columbus, Ohio, US M. JAMES et al. : « Conjugates of phenylacetic acid with taurine and other amino acids in various species »**

**CHEMICAL ABSTRACTS, vol. 89, nr. 15, 9th october 1978, page 113, ref. 123807 p. Columbus, Ohio, US J.R. IDLE et al. : « Taurine conjugates as metabolites of arylacetic acids in the ferret »**

(73) Titulaire : **Société à Responsabilité Limitée dite : B F B Etudes et Recherches Expérimentales**
**9, rue de Nemours**
**F-75011 Paris (FR)**

(72) Inventeur : **Reynaud, Pierre**
**4 Avenue Philippe Bur**
**F-77550 Moissy-Cramayel (FR)**

(74) Mandataire : **Hufenus, Christiane et al**
**CABINET BEAU DE LOMENIE 55, rue d'Amsterdam**
**F-75008 Paris (FR)**

**EP 0 022 022 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Nouveaux dérivés acylés de la taurine et leurs sels et procédé pour leur préparation

L'invention a pour objet de nouveaux dérivés acylés de la taurine répondant à la formule générale suivante :

$$R_1-\underset{\underset{R_2}{|}}{CH}-CO-NH-CH_2-CH_2-SO_3H$$

dans laquelle les groupes $R_1$ et $R_2$ pouvant être identiques ou différents, représentent chacun un groupe alkyle de $CH_3$ à $C_{16}H_{33}$, aryle ou arylalkyle (à l'exclusion des cas où :

a) $R_1$ représente un groupe méthyle et $R_2$ un groupe phényle ;

b) $R_1$ et $R_2$ représentent chacun un groupe phényle)

ainsi que leurs sels, notamment leurs sels avec les aminoacides.

Deux procédés peuvent être utilisés pour préparer ces dérivés : l'acylation par les anhydrides, selon la méthode décrite précédemment pour l'acétyl-taurine (brevet français 77 38 657), c'est-à-dire en milieu anhydre et en l'absence d'alcool, et l'acylation avec les chlorures d'acides appropriés. Pour préparer les dérivés selon l'invention, on fait réagir de préférence le taurinate de potassium avec un chlorure d'acide de formule :

$$R_1 - \underset{\underset{R_2}{|}}{CH} - COCl$$

(dans laquelle $R_1$ et $R_2$ sont définis ci-dessus).

En plus de l'acétyl-taurine, certains dérivés N-alkylacétylés et N-arylacétylés de la taurine ont été préparés antérieurement (Xenobiotica 1978, 8 (4), 253-264). Certaines arylacétyl-taurines ont été retrouvées dans l'urine comme produits d'excrétion des acides arylacétiques, et en particulier de l'acide phénylacétique (Biochem. J. 1971, 124 (2) 15P, 16P).

Mais l'application thérapeutique d'aucun de ces dérivés n'a été décrite jusqu'à maintenant.

La demanderesse a trouvé que les dérivés acylés de la taurine selon l'invention présentent un intérêt comme médicaments ayant notamment une activité neurotrope.

Exemple 1

Préparation de la N(phényléthylacétyl)taurine

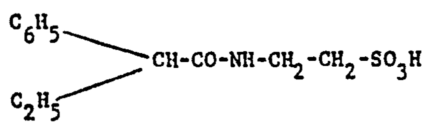 ($C_{12}H_{17}NO_4S$ : P.M.271)

Dans un bécher de 150 ml, on introduit 1,25 g de taurine (0,01 mole) et 22,5 ml de potasse à 10 % (0,04 mole). La solution est maintenue entre 0 et 4 °C et agitée énergiquement. On ajoute goutte à goutte, en 4 h, 3,64 g de chlorure de phényléthyl-acétyle (0,02 mole). Il se forme une fine suspension de gouttelettes qui disparaissent peu à peu. On poursuit l'agitation toute la nuit. Le milieu est alors acidifié en refroidissant vers 0 °C avec HCl jusqu'à pH 1 et extrait trois fois à l'éther ; la phase aqueuse est évaporée à sec sous vide de la trompe à eau et le résidu est épuisé à l'alcool absolu. L'évaporation de ce solvant laisse une huile épaissie qui cristallise par trituration avec l'éther (poids 2,40 g, rendement 88 %). Par recristallisation dans le mélange éthanol-éther, on obtient un produit pur, F. 172-173 °C. La N-phényléthyl-acétyl-taurine est constituée de fins cristaux blancs, stables à l'air ; ils sont solubles dans l'eau, stables à l'air. Ils sont solubles dans le méthanol, l'éthanol, le diméthylsulfoxyde ; insolubles dans l'acétone, l'éther, le chloroforme, le benzène.

Analyse : Calculé % : C 53,13 ; H 6,27 ; N 5,16
Trouvé % : C 53,20 ; H 6,18 ; N 5,10

Exemple 2

Préparation de la N(dipropylacétyl)taurine.

$(CH_3H_7)_2CH—CO—NH—CH_2—CH_2SO_3H$ ($C_{10}H_{21}NO_4S$ ; P.M. 251)

Ce dérivé s'obtient dans les mêmes conditions que le précédent ; ses solubilités sont comparables.

Rendement 50 %, F. 254-255 °C (éthanol-éther). Fins cristaux blancs, non hygroscopiques.

Analyse : Calculé % : C 47,80 ; H 8,36 ; N 5,57
Trouvé % : C 47,91 ; H 8,18 ; N 5,48

Préparation de quelques sels d'arginine et de lysine à partir des acyl-taurines.

Le procédé étant le même pour tous ces sels, on donnera, à titre d'exemple, celui qui permet d'obtenir le N-phényléthylacétyl-taurinate d'arginine.

Exemple 3

Préparation du N-phényléthylacétyl-taurinate de L-arginine.

Dans un ballon rodé de 100 ml, sont introduits 2,710 g de phényléthylacétyl-taurine pure (0,01 mole), 1,742 g de L-arginine (0,01 mole) et 20 ml d'eau distillée. La solubilisation est immédiate et l'on évapore à sec sous pression réduite sans dépasser 45-50 °C ; il reste dans le ballon un sirop très épais que l'on fait cristalliser par grattage avec un peu d'alcool absolu ; la suspension cristalline obtenue est essorée et le produit obtenu lavé plusieurs fois à l'alcool bouillant avant d'être finalement séché (F. 178-179 °C, rendement 85 %).

Le N-phényléthylacétyl-taurinate de L-arginine peut être recristallisé dans le mélange eau + alcool. Il est constitué de cristaux blancs, non hygroscopiques, stables à l'air, solubles dans l'eau, insolubles dans l'éthanol et les solvants organiques.

Le tableau ci-après rassemble quelques propriétés des sels qui ont été obtenus par ce procédé.

Les dérivés acylés de la taurine selon l'invention ont des propriétés pharmacologiques semblables à celles de la taurine, mais à un degré d'activité nettement plus élevé, car ce sont des dérivés plus liposolubles qui pénètrent plus rapidement la barrière hématoméningée. Ils peuvent donc être administrés par la voie orale ou parentérale comme médicaments cardiovasculaires ayant une activité inotrope, antiarythmique ou antihypertensive, ou comme médicaments neurologiques ayant une activité de neurotransmetteurs.

(Voir tableau p. 4)

Tableau

| Nom | P.M. | Rendement produit recristallisé % | Analyses % C H N | Caractères | F°C (instantanée) | Solubilités eau | Solubilités Alcool, solvants organiques |
|---|---|---|---|---|---|---|---|
| N(phényléthylacétyl)-taurinate de L-arginine | 445,2 | 85 | Calculé : 48,52 7,01 17,72<br>Trouvé : 48,35 6,98 17,80 | Cristaux blancs non hygroscopiques | 178-79 | +++ | insoluble |
| N(di-n-propylacétyl)-taurinate de L-arginine | 425,2 | 83 | Calculé : 45,15 8,29 16,45<br>Trouvé : 45,17 8,18 16,29 | " " légèrement hygroscopique | 226-27 | +++ | insoluble |

+++ très soluble

**Revendications**

1. Dérivés acylés de la taurine de formule :

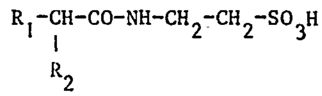

dans laquelle les groupes $R_1$ et $R_2$ pouvant être identiques ou différents représentent chacun un groupe alkyle de $CH_3$ à $C_{16}H_{33}$ ou aryle ou arylalkyle (à l'exclusion des cas où $R_1$ et $R_2$ représentent chacun un groupe phényle et où $R_1$ étant un groupe méthyle, $R_2$ est un groupe phényle) et leurs sels, et en particulier leurs sels avec les aminoacides.

2. Dérivés selon la revendication 1, caractérisés en ce qu'ils consistent en N(phényléthylacétyl)taurine et son sel d'arginine.

3. Dérivés selon la revendication 1, caractérisés en ce qu'ils consistent en N(dipropylacétyl)taurine et son sel d'arginine.

4. Procédé de préparation des dérivés selon l'une des revendications 1 à 3, caractérisé en ce qu'on fait réagir, sur le taurinate de potassium, le chlorure d'acide de formule :

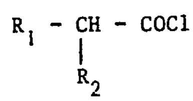

(dans laquelle $R_1$ et $R_2$ sont tels que définis dans la revendication 1).

**Claims**

1. Acylated derivatives of taurine of formula :

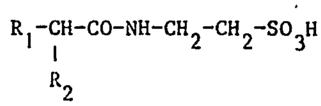

in which one of the two groups $R_1$ and $R_2$ are the same or different and may represent an alkyl group from $CH_3$ to $C_{16}H_{33}$, or aryl or arylalkyl group (except the cases where $R_1$ and $R_2$ each represent a phenyl group and where $R_1$ being a methyl group, $R_2$ is a phenyl group) and salts thereof and in particular their salts with amino acids.

2. The derivative of Claim 1, consisting of N(phenylethylacetyl)taurine and its salt of arginine.

3. The derivative of Claim 1, consisting of N(dipropylacetyl)taurine and its salt of arginine.

4. Process for preparing the derivatives of Claim 1 to 3, characterized in that potassium taurinate is put to react with acid chloride of the formula :

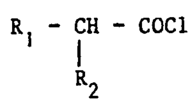

(in which $R_1$ and $R_2$ are defined as in claim 1).

**Ansprüche**

1. Acylierte Taurinderivate der Formel

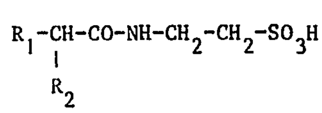

in der die Gruppen $R_1$ und $R_2$ zugleich oder unabhängig jeweils eine Alkylgruppe $CH_3$ bis $C_{16}H_{33}$, eine Arylgruppe oder eine Aralkylgruppe bedeuten, mit Ausnahme der Fälle, in denen $R_1$ und $R_2$ jeweils

Phenyl oder $R_1$ Methyl und $R_2$ Phenyl bedeuten, und ihre Salze, insbesondere ihre Salze mit Aminosäuren.

2. N-(Phenylethylacetyl)-taurin und sein Argininsalz als Taurinderivate nach Anspruch 1.

3. N-(Dipropylacetyl)-taurin und sein Argininsalz als Taurinderivate nach Anspruch 1.

4. Verfahren zur Herstellung der Taurinderivate nach einem der Ansprüche 1 bis 3, gekennzeichnet durch Umsetzung von Kaliumtaurinat mit einem Säurechlorid der Formel

$$R_1 - \underset{\underset{R_2}{|}}{CH} - COCl$$

mit $R_1$ und $R_2$ wie in Anspruch 1.